# EUROPEAN PATENT APPLICATION

(11) **EP 3 289 991 A1**
(43) Date of publication of application: **07.03.2018**
(21) Application number: 16785989.1
(22) Date of filing: 30.04.2016
(51) Int. Cl.: A61B 17/88

(54) **KIRSCHNER WIRE BENDING DEVICE**

(30) Priority: 30.04.2015 CN 201510214954; 16.05.2015 CN 201510248436; 05.08.2015 CN 201510473368
(71) Applicant: Zhengzhou Zezheng Technical Services Ltd., Zhengzhou, Henan 450000 (CN)
(72) Inventor: ZHANG, Weixing, Zhengzhou Henan 450000 (CN); WANG, Xiao, Zhengzhou Henan 450000 (CN); XIAO, Yan, Zhengzhou Henan 450000 (CN)
(74) Representative: Cabinet Beau de Loménie
(86) International application number: PCT/CN2016/080849
(87) International publication number: WO 2016/173561

(57) **Abstract**

A device for bending a kirschner wire comprises: a retaining slot for fixing a kirschner wire, with a contact for applying a bending force to a steel needle fixed by the retaining slot being disposed at a lateral of the retaining slot. Compared with the prior art in which clamping heads are used to clamp a steel needle first and then the steel needle is forcibly bent in the clamped state in most cases, which may be time-consuming and strenuous, the present invention provides not only a device for bending and chipping off an orthopedic steel needle in one time, which is applicable to cases where chipping off of steel needles is required, but also a device capable of only bending a steel needle, which is low in requirement on material and low in manufacturing cost as the force for bending a steel needle is far smaller than the force for chipping off the same, and thus is applicable to cases where only bending of steel needles is required.

## Description

### TECHNICAL FIELD

The present invention relates to a method and a device for forming and chipping off a surgical part, and in particular to bending of an orthopedic kirschner wire and a device for bending and chipping off thereof.

### BACKGROUND ART

Kirschner wires, which are commonly used for setting and fixation of broken bones in orthopedic surgeries, generally have a length of about 20 cm with diameters of different specifications within a range of 0.5 to 4 mm. They are used for fracture fixation without high stress, such as fixation of short and small fractures or avulsion fractures, and also often used in temporary fixation of fracture fragments in orthopedic surgeries. In a surgery, kirschner wires may be clipped according to the sizes of bones to be set, and then inserted into hollowly drilled bones for fixation with exposure of the bent kirschner wires out of the bones, for the purpose of preventing the wire ends left on the surface of the bones from piercing through the skin or fading into the bone surface to be impossibly taken out. At present, wire holders or clamping heads are commonly used for bending, which may be time-consuming and strenuous. The handles disclosed in the patent No. CN87212315U must be rotated for 180 degrees to bend a kirschner wire, and during bending, the side face of a needle holddown block is in contact with the kirschner wire throughout, leading to a great length of the whole bender which is highly inconvenient to use. Consequently, there is no bender capable of bending a kirschner wire at a desired angle in one time at present, let alone use of the bender to further chip off the kirschner wire. Regarding the previous patent this company applied for, a retaining slot is used to limit a kirschner wire, resulting in easy slip of the kirschner wire during bending and chipping off; and when the retaining slot serves as a rotating shaft, it is scarcely possible to further dispose additional clamping parts on the retaining slot due to a very small size of the retaining slot on both sides.

### SUMMARY OF THE INVENTION

The present invention aims at solving the technical problem of continuously applying a clamping force to a kirschner wire during bending or chipping off of the kirschner wire.

A device for bending a kirschner wire comprises a retaining slot for fixing a kirschner wire. A contact for applying a bending force to a steel needle fixed by the retaining slot is disposed at a lateral of the retaining slot.

The retaining slot is long nose pliers and two clamping pieces of a jaw at the head of the long nose pliers; one of the two clamping pieces of the jaw is a forming core block; the contact for applying a bending force to a steel needle fixed by the retaining slot is disposed at a lateral of the retaining slot; the head of the long nose pliers serves as a rotating shaft, and a contact for applying a bending force to a kirschner wire sleeves and rotates around the rotating shaft; and the contact rotates toward the forming core block such that a kirschner wire clamped by the two clamping pieces is bent around the forming core block.

According to the device for bending a kirschner wire, one of the two clamping pieces of the jaw is the forming core block, while the other clamping piece of the jaw is provided with a limiting slot for preventing the slip of a kirschner wire during bending when the kirschner wire is clamped by the jaw limiting slot in match with the forming core block.

According to the device for bending a kirschner wire, a chipping blade of the contact that is used for chipping a kirschner wire and the forming core block form a chipping jaw; and after a kirschner wire is bent by the contact, the chipping blade rotates further around the chipping jaw to chip off the clamped kirschner wire.

According to the device for bending a kirschner wire, the head of the long nose pliers serves as a rotating shaft, and the contact for applying a bending force to a kirschner wire sleeves and rotates around the rotating shaft; stated another way, the head of the long nose pliers, namely the rotating shaft, is made into a rotative surface covered with a sleeve; and the contact is fixed by a rotating part.

According to the device for bending a kirschner wire, the rotative surface forms a cone-shaped contour which is nested in the sleeve matching with the cone-shaped contour, such that the jaw at the head of the long nose pliers can be opened to accommodate a kirschner wire; and the contact is fixed by the sleeve.

The rotative surface forms the cone-shaped contour which is nested in the sleeve matching with the cone-shaped contour, such that the jaw at the head of the long nose pliers can be opened to accommodate a kirschner wire; stated another way, one of the two clamping pieces of the jaw is the rotative surface that forms the cone-shaped contour; and the clamping piece forming the cone-shaped contour is provided with a slotted hole for accommodating the other clamping piece such that the jaw at the head of the long nose pliers can be opened to accommodate a kirschner wire. The slotted hole in the clamping piece forming the cone-shaped contour is in the form of two shaft holes in the position of the rotating shaft, and one shaft hole is formed in the other clamping piece in the position of the rotating shaft; and the rotating shaft runs through the two shaft holes and the one shaft hole.

According to the device for bending and chipping off a kirschner wire, the sleeve is provided with a stop collar for preventing the sleeve from slipping out of a clamping part having a cone-shaped contour during chipping.

The sleeve is circumferentially provided with a through slot, and a limiting column is fastened in a position, corresponding to the through slot, of the clamping piece forming the cone-shaped contour to prevent the sleeve from slipping out of the clamping part having the cone-shaped contour.

The sleeve is circumferentially provided with the through slot, and a limiting slider is fastened in the position, corresponding to the through slot, of the clamping piece forming the cone-shaped contour to prevent the sleeve from slipping out of the clamping part having the cone-shaped contour. According to the device for bending a kirschner wire, the sleeve is provided with a flipping handle which provides bending and chipping-off forces for the contact and the chipping blade thereof. According to the device for bending a kirschner wire, a fixed handle matching with the flipping handle is disposed at a lateral of the long nose pliers, and the flipping handle matches with the fixed handle as a pair of handles to be gripped by hand.

According to the device for bending and chipping off a kirschner wire, the flipping handle is a ratchet wrench, and the position of the sleeve that matches with the ratchet wrench is shaped as a nut matching with the ratchet wrench.

A device for bending akirschner wire comprises a retaining slot. A contact for applying a bending force to a steel needle is disposed above the retaining slot, and the force applied by the contact to a steel needle is a lateral force in a sideways direction of the retaining slot.

The contact applying the lateral force in the sideways direction of the retaining slot to a steel needle means that the contact applies the lateral force to the steel needle in a rectilinear direction forming an included angle with a central axis of the retaining slot.

A bending force is produced between the contact and a corresponding side of an opening of the retaining slot to bend a steel needle.

A limiting pad for bending a steel needle at a desired angle is disposed on one side above the opening of the retaining slot; and the contact and the limiting pad form a chipping jaw to chip off the bent steel needle. A pair of guide rails is disposed between the contact and the retaining slot. The retaining slot is arranged in a frame on which a hydraulic cylinder is fixed, and the contact is fixed by a piston head of the hydraulic cylinder.

The contact is pushed by a cam to achieve bending and chipping.

The retaining slot is arranged in an end face of one clamping head of the jaw, while the contact is arranged at a lateral of an end of the other clamping head, and the two clamping heads are closed to bend a steel needle. A limiting pad for bending a steel needle at a desired angle is disposed on one side above the opening of the retaining slot; and the contact and the limiting pad form the chipping jaw to chip off the bent steel needle. The retaining slot is a movable slot for clamping a steel needle, or a limiting slot fitting with the diameter of a steel needle. The movable slot for clamping a steel needle is another jaw.

In the prior art, clamping heads are used to clamp a steel needle first and then the steel needle is forcibly bent in the clamped state in most cases, which may be time-consuming and strenuous. The present invention provides not only a device for bending and chipping off an orthopedic steel needle in one time, which is applicable to cases where chipping off of steel needles is required, but also a device capable of only bending a steel needle, which is low in requirement on material and low in manufacturing cost as the force of bending a steel needle is far smaller than the force of chipping off the same, and thus is applicable to cases where only bending of steel needles is required.

In the prior art, clamping heads are used to clamp a kirschner wire first and then the steel needle is forcibly bent by using another pliers in the clamped state in most cases, which may be time-consuming and strenuous. In the present invention, a kirschner wire is clamped by using clamping heads first, which conforms to the ordinary clamping practice, and then a bending and chipping mechanism is adopted; that is, the kirschner wire is bent around a forming core block by a flipping block rotating around a rotating shaft to form a desired hook-like bend; if the bent kirschner wire has a proper length, the bending device is removed; if the bent kirschner wire has a great length, further rotation is carried out to chip off the kirschner wire, and the kirschner wire is clamped throughout the chipping process; a limiting slot is arranged at a single side of the clamping part to facilitate slip after bending and chipping off; and a ratchet wrench is arranged, allowing easy bending and chipping off.

### BRIEF DESCRIPTION OF THE DRAWINGS

Fig. 1 is a structural diagram of a clamping part in the present invention in an open state.
Fig. 2 is a structural diagram of the clamping part in the present invention in a closed state with a chipping jaw formed by a chipping blade and a lateral of a forming core block.
Fig. 3 is a diagram showing a kirschner wire being clamped by the clamping part in Fig. 1.
Fig. 4 is a sectional structural diagram showing a cone-shaped sleeve in the present invention sleeving a cone-shaped shaft and the cone-shaped sleeve holding down a stop collar.
Fig. 4-1 is a diagram of an anti-slip structure of a slider.
Fig. 4-2 is a diagram of an exploded anti-slip structure of a slider.
Fig. 4-3 is a diagram of an anti-slip structure of a limiting column.
Fig. 5 is a structural diagram of long nose pliers.
Fig. 6 is a structural diagram showing a sleeve or a cone-shaped sleeve in the present invention holding down a flipping handle.
Fig. 7 is a diagram showing a process of bending a kirschner wire in the present invention.
Fig. 8 is an exploded structural diagram of long nose pliers.
Fig. 9 is a structural diagram of a device capable of only bending in the present invention;
Fig. 10 is a structural diagram of the device capable of only bending with a hydraulic cylinder providing a bending force;
Fig. 11 is a structural diagram of the device capable of only bending with a cam providing a bending force;
Fig. 12 is a structural diagram of the device capable of only bending with a jaw providing a bending force;
Fig. 13 is a structural diagram of a device capable of both bending as well as chipping off in the present invention;
Fig. 14 is a structural diagram of the device capable of both bending as well as chipping off with a hydraulic cylinder providing a bending force;
Fig. 15 is a structural diagram of the device capable of both bending as well as chipping off with a cam providing a bending force;
Fig. 16 is a structural diagram of the device capable of both bending as well as chipping off with a jaw providing a bending force;
Fig. 17 is a structural diagram showing a steel needle being clamped by a screw extending from a lateral of a retaining slot;

### DETAILED DESCRIPTION OF THE EMBODIMENTS

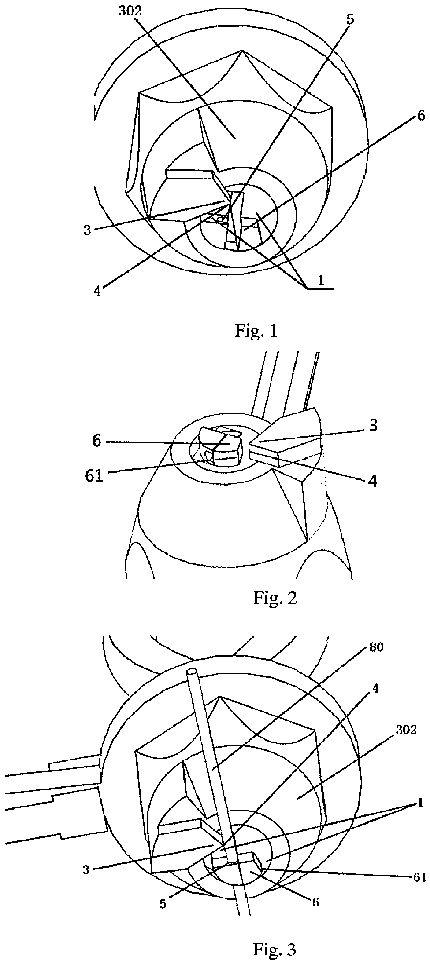

A device for bending and chipping off a kirschner wire, as shown in Fig. 1, Fig. 2 and Fig. 3, comprises a clamping part 1 for clamping a kirschner wire. The clamping part 1 comprises two openable and closeable elements for clamping a kirschner wire. The two openable and closeable elements of the clamping part 1 serve as a rotating shaft. The rotating shaft may be cylindrical or cone-shaped as long as it can allow a contact 3 arranged for applying a bending force to a kirschner wire to rotate around the clamping part 1, i.e., allow the contact 3 to rotate around the rotating shaft. The contact 3 presses down on a kirschner wire such that the kirschner wire is bent around the forming core block 6, and the bending device may be removed after the kirschner wire is bent if the length of pre-clipping of the kirschner wire is appropriate for the length after bending. Alternatively, a large length is reserved after the kirschner wire is bent; a chipping blade 4 of the contact 3 that is used for chipping a kirschner wire and a lateral 61 of the forming core block 6 at one end of the clamping part 1 form a chipping jaw as shown in Fig. 2, and the chipping blade 4 rotates further around the chipping jaw, during which the kirschner wire is chipped, until it comes into contact with the lateral 61 of the forming core block 6 (the distance may also be very short as long as the kirschner wire is chipped off) to chip off the kirschner wire.

According to the device for bending and chipping off a kirschner wire, the clamping part 1 comprises two clamping pieces for clamping a kirschner wire. At the ends of the two clamping pieces, a limiting slot 5 is formed in one clamping piece to prevent the slip of a kirschner wire during chipping. The reason for arranging the limiting slot 5 in only one clamping piece is as follows: a bent kirschner wire 80 may abut on two or three sides of the forming core block 6; for example, as described in the patent No. 2015102149547 to which the present patent claims priority, the bent kirschner wire 80 needs to be pulled away from the forming core block 6 after being chipped off; and if a slot is arranged in the side of the forming core block 6 that is used for clamping the kirschner wire, it may be obstructive for the kirschner wire to break away from the forming core block 6. Certainly, the forming core block 6 may be designed with a lateral stripping slope to facilitate separation of the forming core block 6 from the bent and chipped-off kirschner wire 80.

According to the device for bending and chipping off a kirschner wire, at the ends of the two clamping pieces, the forming core block 6 is disposed on the other clamping piece. After the contact 3 applies a bending force to a kirschner wire to bend the kirschner wire around the forming core block 6, the forming core block 6 serves as a cylinder, and the chipping blade 4 of the contact 3 that is used for chipping a kirschner wire and a lateral of the forming core block 6 form a chipping jaw. The lateral of the forming core block 6 that forms the chipping jaw with the chipping blade 4 may be the intersection line 61 of two sides of the forming core block 6, and may also be an arc surface concentric with the rotation path of the chipping blade 4, for example, the side arc surface of the forming core block 6 as shown in the figure; and the chipping blade 4 rotates further to chip off the kirschner wire. The shape of the forming core block 6 corresponds to that of the bent inner curved surface or curved line of the kirschner wire. The bent inner curved surface or curved line corresponds to the inner countour shape of a bent hook.

Here, as shown in Fig. 1, the two openable and closable elements of the clamping part 1 are opened and a kirschner wire 80 is placed in the opening, and then the two openable and closable elements of the clamping part 1 are closed. As shown in Fig. 3, the kirschner wire 80 is clamped by the corresponding sides of the limiting slot 5 and the forming core block 6 with the contact 3 abutting on the kirschner wire; the contact 3 is rotated to press down on the kirschner wire 80 for bending such that the kirschner wire 80 is bent to a desired bent shape; the chipping blade 4 of the contact 3 and the intersection line 61 of two sides of the forming core block 6 form the chipping jaw. The contact 3 is rotated further, enabling the chipping blade 4 of the contact 3 to chip off the kirschner wire 80. The two openable and closable elements are then opened to allow the forming core block 6 to separate from the bent and chipped-off kirschner wire 80.

According to the device for bending and chipping off a kirschner wire, the outsides of the two clamping pieces form a cone-shaped contour. When the diameters of the ends are identical, the cone-shaped contour can bear a greater torque so that the diameter of the ends can be as small as possible. The cone-shaped contour is nested in a sleeve 302 which is connected to and fix the contact 3. The internal contour of the sleeve 302 matches with the cone-shaped contour formed by the outsides of the two clamping pieces. Certainly, if the outsides of the two clamping pieces form a cylindrical shape, the internal contour of the sleeve 302 is of a matching circular tube shape. Additionally, the external contour of the sleeve 302 may be a cone-shaped contour or a cylindrical contour.

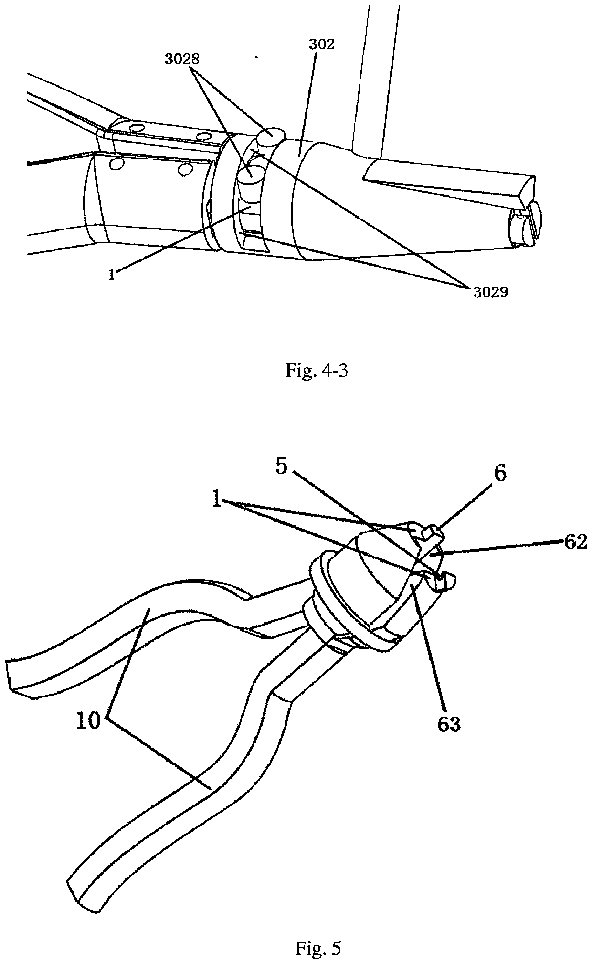

As shown in Fig. 5 to Fig. 8, the rotative surface forms the cone-shaped contour which is nested in the sleeve 302 matching with it, such that the jaw at the head of the long nose pliers can be opened to accommodate a kirschner wire. Stated another way, one of the two clamping pieces of the jaw is the rotative surface that forms the cone-shaped contour, and the clamping piece forming the cone-shaped contour is provided with a slotted hole 62 for accommodating the other clamping piece 63 such that the jaw at the head of the long nose pliers can be opened to accommodate a kirschner wire.

The slotted hole 62 in the clamping piece forming the cone-shaped contour is in the form of two shaft holes 64 in the position of the rotating shaft, and one shaft hole 65 is formed in the other clamping piece in the position of the rotating shaft; and the rotating shaft 66 runs through the two shaft holes 64 and the one shaft hole 65.

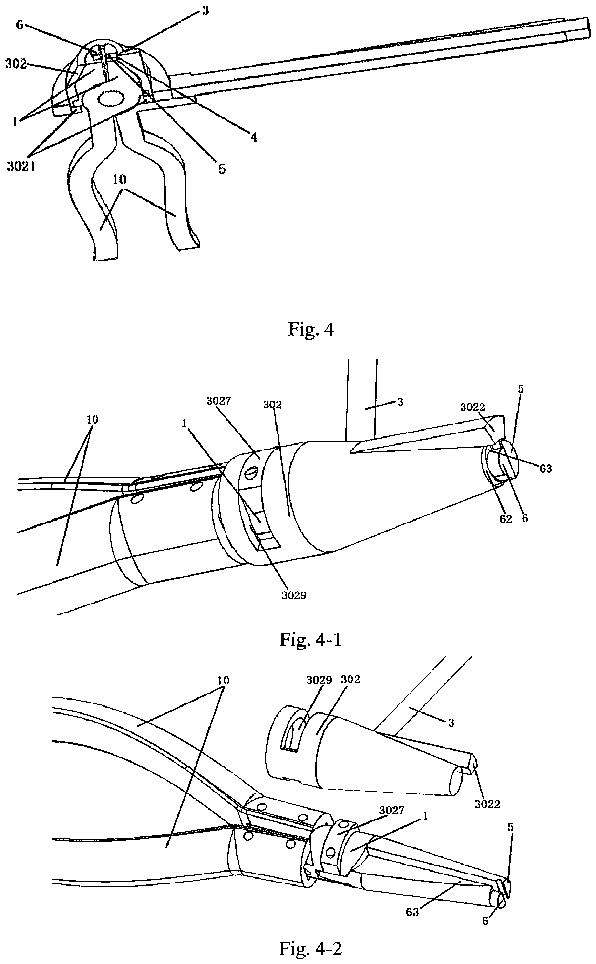

According to the device for bending and chipping off a kirschner wire as shown in Fig. 4, the sleeve 302 is provided with a stop collar 3021. During chipping, the stop collar 3021 and a cone-shaped surface together prevent the sleeve 302 from slipping out of the clamping part 1. Certainly, the clamping part 1 may be externally provided with a ring-shaped lug boss or a ring-shaped slot, and the internal surface of the sleeve 302 matches with the ring-shaped lug boss or the ring-shaped slot to prevent the sleeve 302 from slipping out of the clamping part 1.

As shown in Fig. 4-3, the sleeve 302 is circumferentially provided with a through slot 3029, and a limiting column 3028 is fastened in a position, corresponding to the through slot, of the clamping piece forming the cone-shaped contour to prevent the sleeve 302 from slipping out of the clamping part 1.

As shown in Fig. 4-1 and Fig. 4-2, alternatively, the sleeve 302 is circumferentially provided with the through slot 3029, and a limiting slider 3027 is fastened in a position, corresponding to the through slot, of the clamping piece forming the cone-shaped contour to prevent the sleeve 302 from slipping out of the clamping part 1.

According to the device for bending and chipping off a kirschner wire as shown in Fig. 4 and Fig. 5, the clamping part 1 for clamping a kirschner wire is long nose pliers. Fig. 4 illustrates the sectional shape of the long nose pliers with only one of the two mutually hinged elements of the long nose pliers being shown. A revolving body fitting with the external contour of the mouth of this element is disposed along two ends of the hinge-axis of ordinary long nose pliers with a slot or space for the other hinged element to move; in this way, the two clamping pieces at the head of the long nose pliers are the two clamping pieces of the clamping part 1 that are used for clamping a kirschner wire. The limiting slot 5 at the ends of the two clamping pieces of the jaw cooperates with the forming core block 6 to clamp a kirschner wire with a clamping force being provided by clamping handles 10 of the long nose pliers.

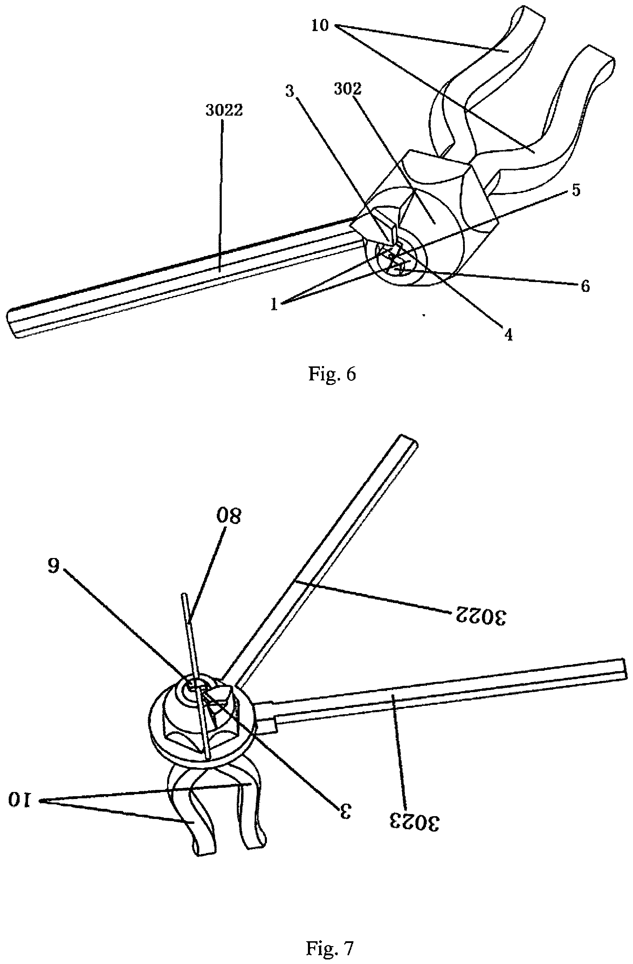

According to the device for bending and chipping off a kirschner wire as shown in Fig. 6, the sleeve 302 is provided with a flipping handle 3022 that provides bending and chipping-off forces for the contact 3 and the chipping blade 4 thereof. In this way, the clamping handles 10 of the long nose pliers provide a clamping force and fix the long nose pliers, while the flipping handle 3022 provides bending and chipping-off forces. Here, it is generally suitable for kirschner wires having 2 mm and less diameters.

According to the device for bending and chipping off a kirschner wire as shown in Fig. 7, a fixed handle 3023 matching with the flipping handle 3022 is disposed at the lateral of the long nose pliers 10. The flipping handle 3022 matches with the fixed handle 3023 to constitute a pair of handles to be gripped by hand. As the flipping handle 3022 matches with the fixed handle 3023 to constitute a pair of handles to be gripped by hand, the clamping handles 10 of the long nose pliers is basically not affected by the bending and chipping-off forces provided by the flipping handle 3022. Here, it is generally suitable for kirschner wires having 2-4 mm diameters.

According to the device for bending and chipping off a kirschner wire, the flipping handle 3022 is a ratchet wrench, and the position of the sleeve 302 that matches with the ratchet wrench is shaped as a nut matching with the ratchet wrench. As the flipping handle 3022 is the ratchet wrench, it is more convenient to use.

A method for bending and chipping a kirschner wire comprises the following steps:
(1) clamping a kirschner wire at an end of a rotating shaft with exposure of the end of the kirschner wire to be bent and chipped;
(2) applying a bending force for rotation around the rotating shaft to the end to be bent and chipped to bend the kirschner wire;
(3) limiting the kirschner wire bent to the desired angle from being bent further, wherein the kirschner wire is limited from being bent further by the end of the rotating shaft; and
(4) continuously applying a chipping force by a blade rotating around the rotating shaft to chip off the kirschner wire.

Reference for the explanations for the method for bending and chipping a kirschner wire in the present invention may be made to the embodiments of the device for bending and chipping off a kirschner wire.

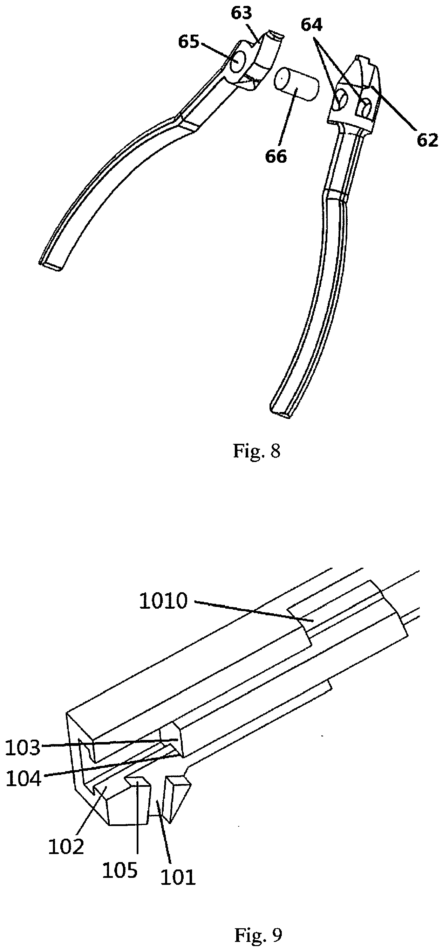

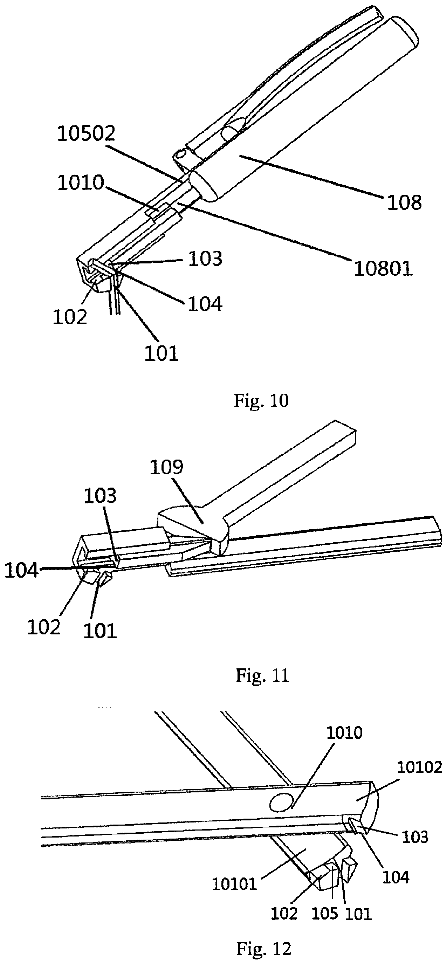

A device for bending a steel needle, as shown in Fig. 9 and Fig. 10, comprises a retaining slot 101 for retaining a steel needle. A contact 103 for applying a bending force to a steel needle is disposed above the retaining slot 1, with a chipping blade 104 for bending a steel needle being arranged on the contact 103. A bending pad 105 for achieving bending of a steel needle to a desired angle is disposed at a side of the retaining slot 101. The right side of the bending pad 105 is at least a distance of the diameter of one steel needle lower than the chipping blade 104 of the contact 103 that moves to stay above the steel needle, and the upper surface of the bending pad 105 is kept at least a distance of the diameter of one steel needle away from the chipping blade 104 that moves to stay thereabove. A bending force is produced by the contact 103 and the corresponding side of the opening of the retaining slot 1, i.e., the contact 103 and the right side of the bending pad 105, to bend a steel needle. With the changing of the steel needle supporting point and the angle of the bending pad, a steel needle may be bent at different angles.

A pair of guide rails 1010 is disposed between the contact 103 and the retaining slot 101, thereby allowing more straight movement of the contact. The retaining slot 1 is arranged in a frame 10502 on which a hydraulic cylinder 108 is fixed, and the contact 103 may be fixed by a piston head 10801 of the hydraulic cylinder 108.

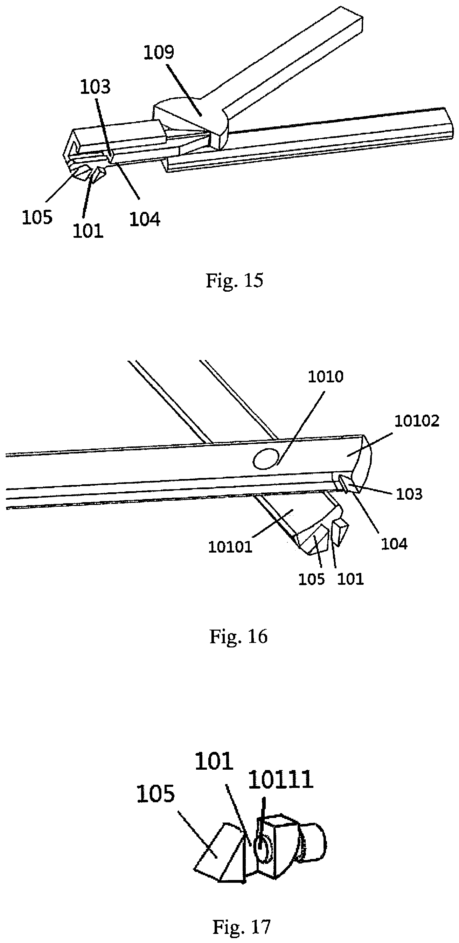

In use of the present invention, a steel needle is retained first, i.e., clamped in the retaining slot fitting with the diameter of the steel needle or in a movable slot as shown in Fig. 17, and thus enabled to resist against a bending force, with exposure of the end to be bent. The contact 103 applying a bending force applies the bending force to the steel needle in a rectilinear direction forming an included angle with the central axis direction of the retaining slot 101. The steel needle is bent when the chipping blade of the contact moves to the protruding steel needle because the right side of the bending pad 105 is lower than the chipping blade; the bent steel needle lies on a limiting pad, and can only be bent further rather than being chipped off by the chipping blade because the left side of the limiting pad is kept a distance of the diameter of one steel needle away from the chipping blade 104 that moves to stay thereabove in height. The device is low in requirement on material and low in manufacturing cost as the force of bending a steel needle is far smaller than the force of chipping off the same, and thus is applicable to cases where only bending of steel needles is required.

As shown in Fig. 11, the contact 103 is pushed by a cam 109 to achieve bending.

As shown in Fig. 12, the retaining slot 101 is arranged in the end face of one clamping head 10101 of the jaw 1010, while the contact 103 is arranged at the lateral of the end of the other clamping head 10102, and the two clamping heads are closed to bend a steel needle with a bending force being applied by the two closed clamping heads to the steel needle in an arc direction.

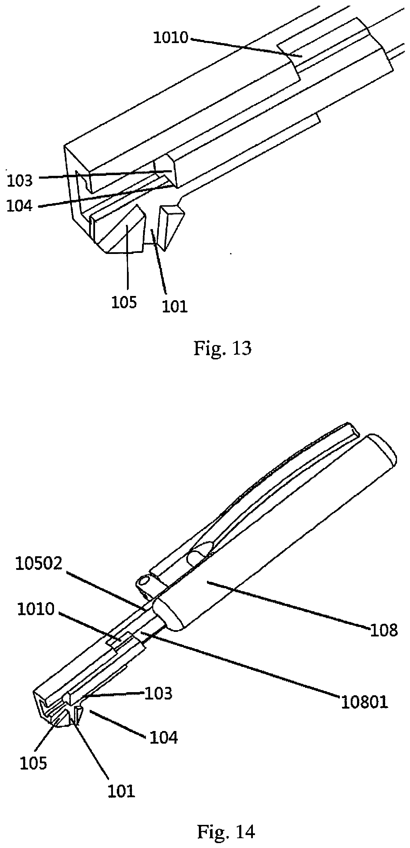

A device capable of bending and chipping off a steel needle, as shown in Fig. 13 to Fig. 16, differs from the device capable of only bending a steel needle as described above as follows: the right side of the bending pad 105 is at least a distance of the diameter of one steel needle lower than the chipping blade 104 of the contact 103 that moves to stay above the steel needle; the left side of the bending pad 105 is connected to the limiting pad 102, and the upper surface of the limiting pad 102 comes into contact with the cutting blade that moves to stay thereabove to form a chipping jaw for chipping off the bent steel needle; and with the changing of the steel needle supporting point and the angle between the bending pad and the limiting pad, the steel needle may be bent at different angles and chipped off. Bending and chipping a steel needle by using the present invention may include the following steps:
(1) clamping a steel needle with exposure of the end of the steel needle to be bent and chipped;
(2)applying a bending force to the end to be bent and chipped to bend the steel needle;
(3) limiting the steel needle bent to the desired angle from being bent further; and
(4) continuously applying a chipping force by the chipping blade to chip off the steel needle.

Specifically, a steel needle is retained first (i.e., clamped in the retaining slot fitting with the diameter of the steel needle or in the movable slot as shown in Fig. 17, and thus enabled to resist against a bending force), with exposure of the end to be bent. The contact 103 applies a lateral force in the sideways direction of the retaining slot to the steel needle to bend it. The bent steel needle lies on the limiting pad 105, and the chipping blade 104 of the contact 103 continuously applies a chipping force to chip off the steel needle.

As shown in Fig. 13, Fig. 14 and Fig. 15, the contact applying a bending force applies the bending force to the steel needle in a rectilinear direction forming an included angle with the central axis direction of the retaining slot, with the chipping force being continuation of the bending force in the rectilinear direction.

As shown in Fig. 16, the steel needle is retained in one clamping head 10101 of two clamping heads. The bending force in step 2 is the force in an arc direction applied by the lateral of the end of the other clamping head 10102 to the steel needle when closed, and the force applied continuously in step 4 is in a direction as continuation of the force in the arc direction in step 2.

The retaining slot 101 is a movable slot for clamping a steel needle; in other words, a sidewall of the slot is capable of movable clamping. A sidewall of the slot in Fig. 17 extends out of the end face 10111 where a bolt bar is screwed down to clamp a steel needle, thus preventing the steel needle from moving or slipping out of the limiting slot during bending and chipping.

In an embodiment of closing two clamping heads to bend a steel needle, the movable slot for clamping the steel needle may also be another jaw; stated another way, two sidewalls of the movable slot are arranged in one clamping head, and a bending contact is arranged on the other clamping head, and the two clamping heads form a jaw that may be closed to bend or chip off a steel needle. The another jaw may be arranged to clamp the two sidewalls of the movable slot to enable the two sidewalls to clamp a steel needle for bending and chipping off.

Alternatively, a limiting slot fitting with the diameter of a steel needle is also possible. When a steel needle is under a bending force and a chipping force, the limiting slot may limit the steel needle from slipping out therefrom. The limiting slot fitting with the diameter of a steel needle is illustrated in all of the other figures except Fig. 17 in the drawings accompanying the description.

## Claims

1. A device for bending a kirschner wire, comprising: a retaining slot for fixing a kirschner wire, with a contact for applying a bending force to a steel needle fixed by the retaining slot being disposed at a lateral of the retaining slot.

2. The device for bending a kirschner wire of claim 1, wherein the retaining slot is long nose pliers and two clamping pieces of a jaw at the head of the long nose pliers; one of the two clamping pieces of the jaw is a forming core block (6); the contact for applying a bending force to a steel needle fixed by the retaining slot is disposed at a lateral of the retaining slot; the head of the long nose pliers serves as a rotating shaft, and a contact (3) for applying a bending force to a kirschner wire sleeves and rotates around the rotating shaft; and the contact (3) rotates toward the forming core block (6) such that a kirschner wire clamped by the two clamping pieces is bent around the forming core block (6).

3. The device for bending a kirschner wire of claim 1 or 2, wherein one of the two clamping pieces of the jaw is the forming core block (6), while the other clamping piece of the jaw is provided with a limiting slot (5) for preventing slip of a kirschner wire during bending when the kirschner wire is clamped by the jaw limiting slot (5) in match with the forming core block (6).

4. The device for bending a kirschner wire of claim 3, wherein a chipping blade (4) of the contact (3) that is used for chipping a kirschner wire and the forming core block (6) form a chipping jaw; and after a kirschner wire is bent by the contact (3), the chipping blade (4) rotates further around the chipping jaw to chip off the clamped kirschner wire.

5. The device for bending a kirschner wire of claim 4, wherein the head of the long nose pliers serves as a rotating shaft, and the contact (3) for applying a bending force to a kirschner wire sleeves and rotates around the rotating shaft; stated another way, the head of the long nose pliers, namely the rotating shaft, is made into a rotative surface covered with a sleeve (302); and the contact (3) is fixed by a rotating part.

6. The device for bending a kirschner wire of claim 5, wherein the rotative surface forms a cone-shaped contour which is nested in the sleeve (302) matching with the cone-shaped contour, such that the jaw at the head of the long nose pliers can be opened to accommodate a kirschner wire; and the contact (3) is fixed by the sleeve (302).

7. The device for bending a kirschner wire of claim 6, wherein the rotative surface forms the cone-shaped contour which is nested in the sleeve (302) matching with the cone-shaped contour, such that the jaw at the head of the long nose pliers can be opened to accommodate a kirschner wire; stated another way, one of the two clamping pieces of the jaw is the rotative surface that forms the cone-shaped contour; and the clamping piece forming the cone-shaped contour is provided with a slotted hole (62) for accommodating the other clamping piece (63) such that the jaw at the head of the long nose pliers can be opened to accommodate a kirschner wire.

8. The device for bending a kirschner wire of claim 7, wherein the slotted hole (62) in the clamping piece forming the cone-shaped contour is in the form of two shaft holes (64) in the position of the rotating shaft, and one shaft hole (65) is formed in the other clamping piece in the position of the rotating shaft; and the rotating shaft (66) runs through the two shaft holes (64) and the one shaft hole (65).

9. The device for bending a kirschner wire of claim 8, wherein the sleeve (302) is provided with a stop collar (3021) for preventing the sleeve (302) from slipping out of a clamping part (1) having a cone-shaped contour during chipping.

10. The device for bending a kirschner wire of claim 8, wherein the sleeve (302) is circumferentially provided with a through slot (3029), and a limiting column (3028) is fastened in a position, corresponding to the through slot (3029), of the clamping piece forming the cone-shaped contour to prevent the sleeve (302) from slipping out of the clamping part (1) having the cone-shaped contour.

11. The device for bending a kirschner wire of claim 8, wherein the sleeve (302) is circumferentially provided with the through slot (3029), and a limiting slider (3027) is fastened in the position, corresponding to the through slot (3029), of the clamping piece forming the cone-shaped contour to prevent the sleeve (302) from slipping out of the clamping part (1) having the cone-shaped contour.

12. The device for bending a kirschner wire of claim 8, wherein the sleeve (302) is provided with a flipping handle (3022) which provides bending and chipping-off forces for the contact (3) and the chipping blade (4) thereof.

13. The device for bending and chipping off a kirschner wire of claim 12, wherein a fixed handle (3023) matching with the flipping handle (3022) is disposed at a lateral of the long nose pliers (10), and the flipping handle (3022) matches with the fixed handle (3023) as a pair of handles to be gripped by hand.

14. The device for bending a kirschner wire of claim 13, wherein the flipping handle (3022) is a ratchet wrench, and the position of the sleeve (302) that matches with the ratchet wrench is shaped as a nut matching with the ratchet wrench.

15. The device for bending a kirschner wire of claim 1, wherein a contact (103) for applying a bending force to a steel needle is disposed above the retaining slot (101), and the force applied by the contact (103) to a steel needle is a lateral force in a sideways direction of the retaining slot (101).

16. The device for bending a kirschner wire of claim 15, wherein the contact (103) applying the lateral force in the sideways direction of the retaining slot (101) to a steel needle means that the contact (103) applies the lateral force to the steel needle in a rectilinear direction forming an included angle with a central axis of the retaining slot (101).

17. The device for bending a kirschner wire of claim 16, wherein a bending force is produced between the contact (103) and a corresponding side of an opening of the retaining slot to bend a steel needle.

18. The device for bending a kirschner wire of claim 17, wherein a limiting pad (102) for bending a steel needle at a desired angle is disposed on one side above the opening of the retaining slot; and the contact and the limiting pad form a chipping jaw to chip off the bent steel needle.

19. The device for bending a kirschner wire of claim 17, wherein a pair of guide rails (1010) is disposed between the contact (103) and the retaining slot (101).

20. The device for bending a kirschner wire of claim 19, wherein the retaining slot (101) is arranged in a frame (10502) on which a hydraulic cylinder (108) is fixed, and the contact (103) is fixed by a piston head (10801) of the hydraulic cylinder (108).

21. The device for bending a kirschner wire of claim 19, wherein the contact (103) is pushed by a cam (109) to achieve bending and chipping.

22. The device for bending a kirschner wire of claim 15, wherein the retaining slot (101) is arranged in an end face of one clamping head (10101) of the jaw (1010), while the contact (103) is arranged at a lateral of an end of the other clamping head (10102), and the two clamping heads are closed to bend a steel needle.

23. The device for bending a kirschner wire of claim 22, wherein the limiting pad (102) for bending a steel needle at a desired angle is disposed on one side above the opening of the retaining slot; and the contact (103) and the limiting pad form the chipping jaw to chip off the bent steel needle.

24. The device for bending a kirschner wire of claim 15, wherein the retaining slot (101) is a movable slot for clamping a steel needle, or a limiting slot fitting with the diameter of a steel needle.

25. The device for bending a kirschner wire of claim 22 or 24, wherein the movable slot for clamping a steel needle is another jaw.
